## Europäisches Patentamt
### European Patent Office
### Office européen des brevets

(11) Veröffentlichungsnummer: **0 151 414**
**A2**

(12) **EUROPÄISCHE PATENTANMELDUNG**

(21) Anmeldenummer: **85100498.6**

(22) Anmeldetag: **18.01.85**

(51) Int. Cl.⁴: $A\ 47\ C\ 21/06$

(30) Priorität: **19.01.84 DE 8401477 U**
**17.11.84 DE 8433836 U**

(43) Veröffentlichungstag der Anmeldung:
**14.08.85 Patentblatt 85/33**

(84) Benannte Vertragsstaaten:
**AT BE CH DE FR GB IT LI LU NL SE**

(71) Anmelder: **Planeta Hausgeräte GmbH & Co.**
**Elektrotechnik KG**
**Georgenstrasse 11-13**
**D-8948 Mindelheim(DE)**

(72) Erfinder: **Neumann, Hans**
**Grüntenstrasse 3**
**D-8949 Stetten(DE)**

(74) Vertreter: **Riebling, Günter, Dr. et al,**
**Patentanwälte Dr.-Ing., Dipl.-Ing., Ing.(grad) Günter**
**Riebling Dr.-Ing., Dipl.-Ing. Peter Riebling Rennerle 10**
**Postfach 3160**
**D-8990 Lindau (Bodensee)(DE)**

(54) Betteinlage.

㉟ Es werden zwei unterschiedliche Ausführungsformen einer Betteinlage beschrieben, von denen die eine (1) aus einer von einem Schaumkunststoff (2-4) umschlossenen Metallfolie (5-8) besteht, die eine hochpermeable Nickellegierungsfolie ist, während die andere Ausführungsform einer Betteinlage aus einer aus gewirkter Schafschurwolle bestehende Außenschicht (3) aufweist, und die gegenüberliegende Außenschicht aus einem Trikotstoff (5) aus Baumwolle besteht, in welche Kupferfäden (1) voneinander isoliert angeordnet sind.

FIG 1

---

Betteinlage

---

Gegenstand der vorliegenden Erfindung ist eine Betteinlage nach dem Oberbegriff des Patentanspruchs 1.

Eine derartige Betteinlage ist beispielsweise mit dem Gegenstand des Gebrauchsmusters 6 904 160 bekannt geworden, bei der als Metallfolie eine Aluminiumfolie verwendet wird. Diese Aluminiumfolie soll gegen elektromagnetische Strahlen jedweder Art schützen, kann diesen Zweck aber nicht erfüllen, weil eine derartige Aluminiumfolie für Magnetfelder durchlässig ist.

Mit dem Gegenstand des Gebrauchsmusters G 83 o2 74o.8 ist eine Untermatratze bekannt geworden, bei der ebenfalls eine Aluminiumfolie verwendet wird, welche die gleichen, oben genannten Nachteile aufweist.

Der vorliegenden Erfindung liegt die Aufgabe zugrunde, eine Betteinlage der eingangs genannten Art so weiterzubilden, daß sie gegen die von der Unterseite, z.B. vom Erdboden her wirkenden, elektromagnetischen, Felder schützt.

Zur Lösung der gestellten Aufgabe ist die Erfindung dadurch gekennzeichnet, daß die Betteinlage eine von Schaumkunststoff umschlossene Metallfolie aufweist, welche sich über die gesamte Fläche der Betteinlage erstreckt, und daß die Metallfolie eine hochpermeable Nickellegierungsfolie (Mu-Metall) ist.

Eine derartige Nickellegierungsfolie wird unter der Handelsbezeichnung Mu-Metall-Folie vertrieben und zeichnet sich dadurch aus, daß sie hochpermeabel ist und dadurch nicht durchlässig für elektromagnetische Felder mit hohem

magnetischen Feldanteil ist. Dieser Effekt beruht darauf daß die entsprechend eingestrahlten Magnetfelder zu Wirbelströmen in der Nickellegierungsfolie führen, die den eingestrahlten Magnetfeldern entgegengesetzt sind, so daß an der gegenüberliegenden Seite der Nickellegierungsfolie ein Magnetfeld nicht mehr meßbar ist.

Eine Abschirmung des ansonsten vorhandenen Erdmagnetfeldes wird durch die neuerungsgemäss vorgeschlagene Betteinlage jedoch in vorteilhafter Weise nicht erreicht, weil das Erdmagnetfeld auch von oben her auf die auf der Betteinlage schlafenden Personen einstrahlt und die von oben her auf den Schläfer einwirkenden elektromagnetischen Felder des Erdmagnetfeldes lediglich durch die eingelegte Metallfolie umgelenkt werden.

Ein derartiges Erdmagnetfeld ist erwünscht und soll beibehalten werden.

Bei den Metallfolie nach dem eingangs genannten Stand der Technik besteht der Nachteil, daß diese Metallfolien keinerlei Luftdurchgang zeigen, so daß sie in der Verwendung als Betteinlage (z.B. als Untermatratze) zu einer Feuchte-Akkumulation bei den darüberliegenden Geweben führen und auf die Dauer es hierdurch zu ungesunden und feuchten Bettklimata kommt.

Dies wird erfindungsgemäss nach dem Gegenstand des Anspruches 3 dadurch vermieden,daß die Metallfolie in sich über die Breite der Betteinlage erstreckende Streifen aufgeteilt ist, daß die Streifen in unterschiedlichen, horizontalen Ebenen in der Betteinlage angeordnet sind und daß die Streifen der unterschiedlichen Ebenen sich schuppenartig überlappen.

Auf diese Weise wird ein optimaler Feuchtedurchgang durch die Betteinlage erreicht, weil durch die schuppenartige Überlappung der in Streifen aufgeteilten Metallfolien ein guter Feuchtigkeits- und Luftdurchgang durch
die Matratze gewährleistet ist, vorausgesetzt, daß die
übrigen Materialien der Betteinlage ebenfalls entsprechend
der vorliegenden Erfindung feuchtigkeits- und luftdurchlässig sind.

Eine derartige Betteinlage kann entweder als Oberbett,
d.h. auf der Oberseite einer Matratze verwendet werden,
als auch in einer anderen Ausführungsform als Unterlage
für eine Matratze, d.h. eine solche Betteinlage wird nach
der zweiten Ausführungsform unmittelbar auf den Lattenrost des Bettes gelegt und die Matratze wird darüber gelegt.

Es wird hierbei bevorzugt, wenn der Überlappungsbereich
etwa 1 cm beträgt. Auf diese Weise ist sichergestellt,
daß die Metallfolie noch "magnetisch dicht" ist und
trotzdem ein optimaler Luft- und Feuchtedurchgang durch
die Betteinlage gewährleistet ist.

Eine besonders einfache Befestigung der streifenartigen
Metallfolien ergibt sich dadurch, daß die Streifen der
Metallfolie jeder Ebene in den Taschen jeweils eines
Tunnelgewebes auf Lücke angeordnet sind. D.h., es wird
ein Tunnelgewebe bevorzugt aus einem Baumwoll-Trikot
Gewebe verwendet, in denen streifenförmige Taschen eingenäht sind und in diese Taschen, welche gegeneinander
durch Nähte getrennt sind, werden die streifenartigen
Metallfolien eingeschoben.

Auf diese Weise ist eine sehr schnelle und einfache
Montage der Betteinlage möglich, denn ein solches Tunnelgewebe erstreckt sich über die gesamte Ausdehnung der
Betteinlage und enthält die in Streifen aufgeteilten
Metallfolien. Ein derartiges Tunnelgewebe wird dann einfach auf eine Schaumstoffschicht aufgelegt und von einer weiteren Schaumstoffschicht nach oben hin abgedeckt.

Es wird hierbei folgender Aufbau der Betteinlage besonders bevorzugt:

Die Oberseite wird gebildet von einer Deckschicht, bevorzugt aus einem reinen Seidengewebe, das zur Unterseite hin mit einem Faservlies aus Schafschurwolle abgedeckt ist.

Darunter wird eine erste Schaumstoffschicht angeordnet, an deren Unterseite das Tunnelgewebe mit der ersten horizontalen Schicht von streifenförmig aufgeteilten Metallfolien angeordnet ist.

Dieses Tunnelgewebe wird an der Unterseite hin durch eine weitere Schaumstoffschicht abgedeckt, an deren Unterseite die zweite Ebene des Tunnelgewebes mit den darin angeordneten, streifenförmigen Metallfolien angeordnet ist.

Die Unterseite dieses Tunnelgewebes wird wiederum von einer Schaumstoffschicht abgedeckt, an deren Unterseite wiederum das Faservlies aus Schafschurwolle und die darüber angeordnete Deckschicht besteht aus einem gewebten oder gewirkten Vliesstoff.

Der gesamte Aufbau der Betteinlage zeichnet sich dadurch aus, daß nur Naturprodukte verwendet werden, und solche Produkte, die einen ausgezeichneten Feuchte- und Luftdurchgang gewährleisten.

Auf diese Weise wird ein angenehmes Bettklima im Schlaf erzeugt und die schlafende Person ist von unten her optimal gegen die Einstrahlung elektromagnetischer Strahlen geschützt.

Dank der schuppenartigen Anordnung der Metallfolien in unterschiedlichen Ebenen wird gewährleistet, daß die Betteinlage selbst sehr flexibel ist und sich den Körperbewegungen und der Körperform des Schläfers optimal anpasst.

Zur Lösung der gleichen Aufgabe dient die im folgenden beschriebene weitere Ausführungsform einer Betteinlage, die im wesentlichen aus einem Textilstoff besteht, in den Kupferfäden eingewirkt oder eingewebt sind.

Derartige Textilstoffe wurden auch für Unterbetten und Kleidungsstücke verwandt. Die eingewebten Kupferfäden sollten nach Art eines faradayischen Käfigs ein in sich leitendes Gewebe bilden, zur Abschirmung von elektromagnetischen Feldern. Bei der Verwendung eines derartigen Stoffes für ein Unterbett wurde sogar dafür gesorgt, daß die Drähte bzw. das Gewebe unter sich leitend verbunden waren, und auch noch eine leitende Verbindung zu dem genauso ausgebildeten Gewebe des Oberbettes vorhanden war.

Nach der Erfindung sollen nicht elektrostatische und/oder elektromagnetische Felder abgeschirmt werden, sondern es soll eine Abschirmung gegenüber Erdstrahlen erfolgen. Erdstrahlen lassen sich nicht in die Gesetzmässigkeit elektrostatischer und elektromagnetischer Felder ein - ordnen. Hier ist man auf Versuch angewiesen, z.B. durch Pendeln oder Wünschelruten kann man feststellen, ob Erdstrahlen, in der Regel herrührend von unterirdischen Wasseradern, abgeschirmt oder nicht abgeschirmt werden. Man hat sich hierbei der verschiedensten Materialien bedient, wie Quarz, Glas, Kupferspiralen und ähnliches.

Bei der Erfindung wird hier ein ganz anderer Weg beschritten, der sich als erfolgreich erwiesen hat.

Die Lösung der Aufgabe nach der Erfindung, eine Abschirmung gegen Erdstrahlen - insbesondere beim schlafenden Menschen im Bett zu erreichen - besteht darin, daß bei einem Unterbett die eine Außenschicht von einem Schafwirkpelz, gewirkt aus 100%-iger Schafschurwolle, besteht, darüber ein Wollvlies 4, aus 100%-iger Schafschurwolle, bestehend folgt und als andere Außenschicht ein Trikotstoff 5 aus 100%-iger Baumwolle vorhanden ist, in den Kupferfäden voneinander isolierend, z.B. eingewirkt oder eingewebt sind.

Der Schwerpunkt liegt hier darauf, daß die Kupferfäden voneinander isolierend angeordnet sind.

Ein bevorzugtes Ausführungsbeispiel bei der Anwendung der Erfindung bei einem Unterbett besteht darin, daß die Kupferfäden zueinander parallel und voneinander isolierend verlaufend eingewebt und/oder eingewirkt sind.

Besonders zweckmässig hat es sich noch erwiesen, daß die Kupferfäden zu den Längsstreifen des Unterbettes 8 parallel verlaufen.

Zweckmässig ist ferner, daß die Kupferfäden einen Durchmesser von 0,6 bis 1.0 mm besitzen und im Abstand von 5 bis 15 mm voneinander angeordnet verlaufen.

Eine weitere mögliche Ausführung besteht darin, daß die Kupferfäden von mindestens zwei miteinander verdrillten Kupferfäden gebildet sind.

Will man diesen Textilstoff auch für Kleidungsstücke, insbesondere Unterwäsche, anwenden, besteht ein weiteres Merkmal darin, daß der Textilstoff als Kleidungsstück, z.B. als Unterwäsche, verarbeitet ist und die Kupferfäden zueinander parallel und voneinander isoliert verlaufend angeordnet sind.

Es ist also wesentlich, daß die Kupferfäden voneinander isoliert sind, und zwangsläufig dann auch zueinander parallel verlaufen. Selbstverständlich wäre es auch möglich, daß,wenn z.B. die Kupferfäden auf der einen Außenseite des Unterbettes in Längsrichtung zum Unterbett verlaufen, sie auf der gegenüberliegenden Seite quer zur Längsrichtung verlaufen können, sofern immer dafür gesorgt ist, daß die Drähte voneinander isoliert sind, z.B. durch entsprechende Zwischenlagen.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.
Alle in den Unterlagen offenbarten Angaben und Merkmale, insbesondere die in den Zeichnungen dargestellte, räumliche Ausbildung werden als erfindungswesentlich beansprucht, soweit sie einzeln oder in Kombination gegenüber dem Stand der Technik neu sind.

Im folgenden wird die Erfindung anhand von lediglich einen Ausführungsweg darstellende Zeichnung näher erläutert. Hierbei gehen aus den Zeichnungen und ihrer Beschreibung weitere wesentliche Merkmale und Vorteile der Neuerung hervor.

Es zeigen:

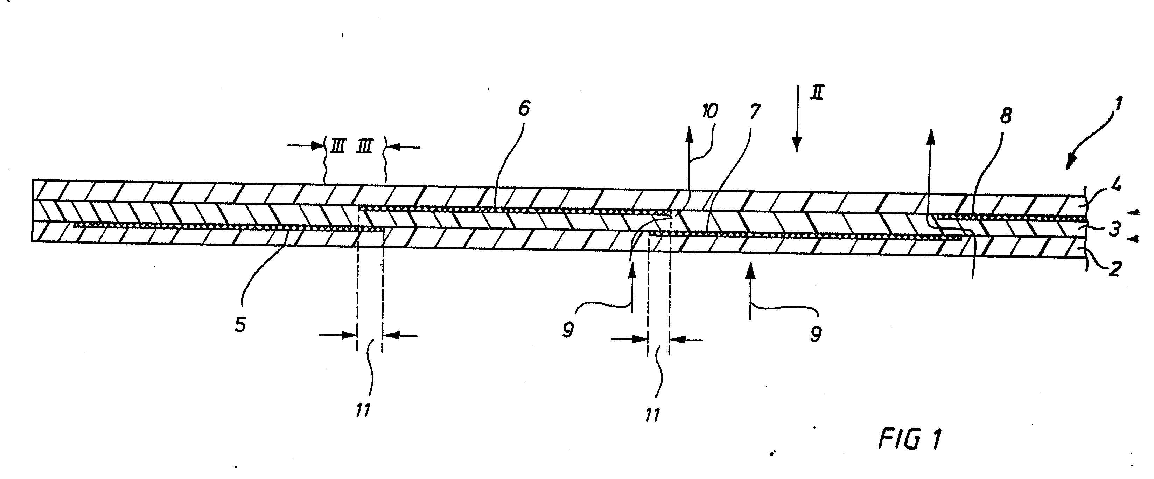

Figur 1: Längsschnitt durch eine Betteinlage gemäss Linie I-I in Figur 2,

Figur 2: Draufsicht in Richtung des Pfeiles II in Figur 1,

Figur 3: ein vergrößerter Teilschnitt durch die Bett - einlage gemäss der Linie III-III in Figur 1,

Figur 4 zeigt einen Schnitt durch ein Unterbett nach einer weiteren Ausführungsform,

Figur 5 ist eine Draufsicht in schematischer Form auf die Figur 4,

Figur 6 zeigt als Einzelheit die Möglichkeit, Kupferfäden durch Verdrillen einzelner Fäden herzustellen.

Die Betteinlage 1 weist drei lose übereinandergelegte Schaumstoffschichten 2,3,4 etwa gleicher Dicke auf. Es wird hierbei ein offenzelliger Schaumstoff bevorzugt, der einen guten Luft- und Feuchtetransport gewährleistet.

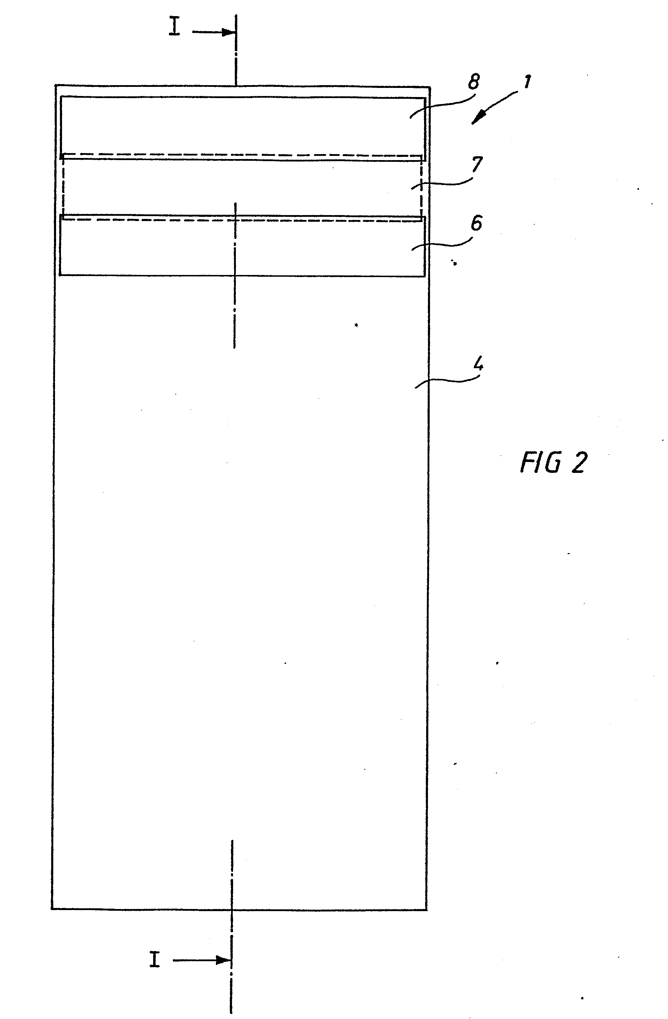

Auf der Oberseite der mittleren Schaumstoffschicht 3 sind in gegenseitigem Abstand, sich über die Breite der Betteinlage (vgl. Figur 2) erstreckende Streifen einer Metallfolie 6,8 angeordnet.

Im Zwischenraum zwischen der Unterseite der mittleren Schaumstoffschicht 3 und der Oberseite der unteren Schaumstoffschicht 2 sind weitere Streifen der Metallfolie 5,7 angeordnet und zwar so, daß die Metallstreifen der unteren Ebene 20 die Lücken der oberen Ebene 19 abdecken und einen Überlappungsbereich 11 bilden, der bevorzugt etwa 1 cm in Richtung der Längsachse der Betteinlage beträgt.

Die in der oberen Ebene 19 angeordneten Metallfolien 6,8 sind damit genauso wie die in der unteren Ebene 20 angeordneten Metallfolien 5,7 von allen Seiten von den Schaumstoffschichten 2,3,4 umgeben.

Lästige Knittergeräusche beim Belasten dieser Metallfolien 5 - 8 werden damit vermieden und dennoch wird ein guter Luft- und Feuchtetransport durch die Betteinlage in Pfeilrichtung 9 und in Gegenrichtung hierzu gewährleistet.

Die von unten in Pfeilrichtung 9 einströmende Luft durchströmt den Überlappungsbereich 11 zwischen den im Abstand einander gegenüberliegenden Metallfolien 5,6 bzw. 7,6 bzw. 7,8. Die Luft kann daher in Pfeilrichtung 10 aus der Oberseite der Betteinlage entströmen. In umgekehrter Richtung erfolgt hierbei der Feuchtetransport während des Schlafes.

Wegen der schuppenartig übereinander angeordneten und einen gegenseitigen Abstand aufweisenden Metallfolien 5 - 8 wird eine ausgezeichnete.magnetische Abschirmung (wegen der Überlappungsbereiche 11) erreicht und trotzdem wird ein guter Feuchte- und Lufttransport durch die Betteinlage 1 gewährleistet.

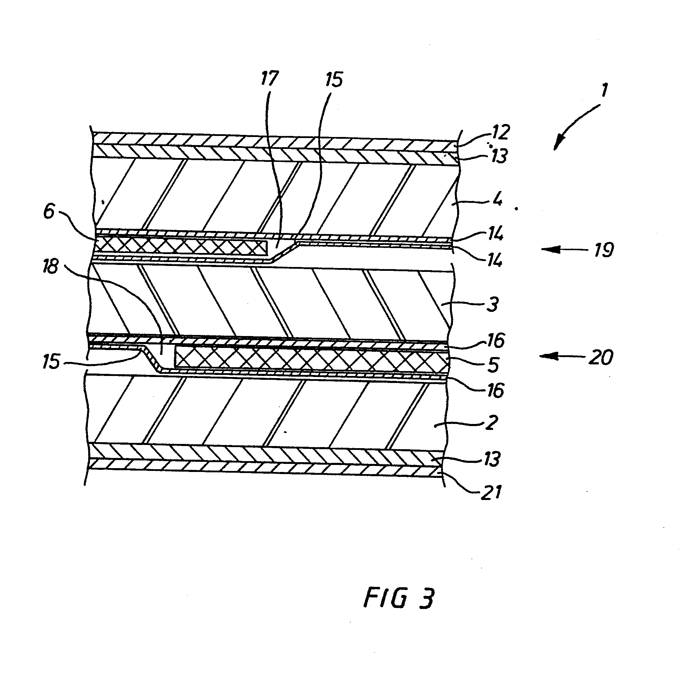

Ein besonders einfacher Aufbau und eine einfache Montage ergeben sich bei einem Aufbau der Betteinlage 1 gemäss der Figur 3. Eine obere Deckschicht 12 besteht aus einem Seidengewebe, welches an der Unterseite von einer Polsterschicht 13 abgedeckt ist, bevorzugt aus einem Vlies aus Schafschurwolle besteht. Darunter ist die erste Schaumstoffschicht 4 angeordnet, welche mit der darunterliegenden Schaumstoffschicht 3 einen Zwischenraum bildet, in dem ein Tunnelgewebe 14 angeordnet ist. Dieses Tunnelgewebe 14 besteht aus zwei Schichten eines Trikot-Gewebes, in welches mit Hilfe von senkrecht zur Zeichenebene der Figur 3 angeordneten Nähten 15 in gegenseitigem Abstand Taschen 17 angeordnet sind, in welche jeweils eine Metallfolie 6 eingelegt ist. Hierdurch erfolgt eine optimale Befestigung der Metallfolien 5 - 8, nachdem diese lose .in den Taschen 17,18 des jeweiligen Tunnelgewebes 14,16 aufgenommen sind.

Ein gleicher Aufbau ergibt sich im Zwischenraum zwischen der mittleren Schaumstoffschicht 3 und der unteren Schaumstoffschicht 2, weil dort ebenfalls ein Tunnelgewebe 16 mit durch Nähte 15 abgesteppte Taschen 18 vorgesehen ist.

An der Unterseite der unteren Schaumstoffschicht 2 ist wiederum eine Polsterschicht 13 aus einem Vlies aus Schafschurwolle angeordnet, welches nach unten hin durch einen Baumwoll-Vliesstoff 21 abgedeckt ist.

Alle Materialien der Betteinlage 1 sind Naturstoffe, die ein angenehmes Schlafklima gewährleisten.

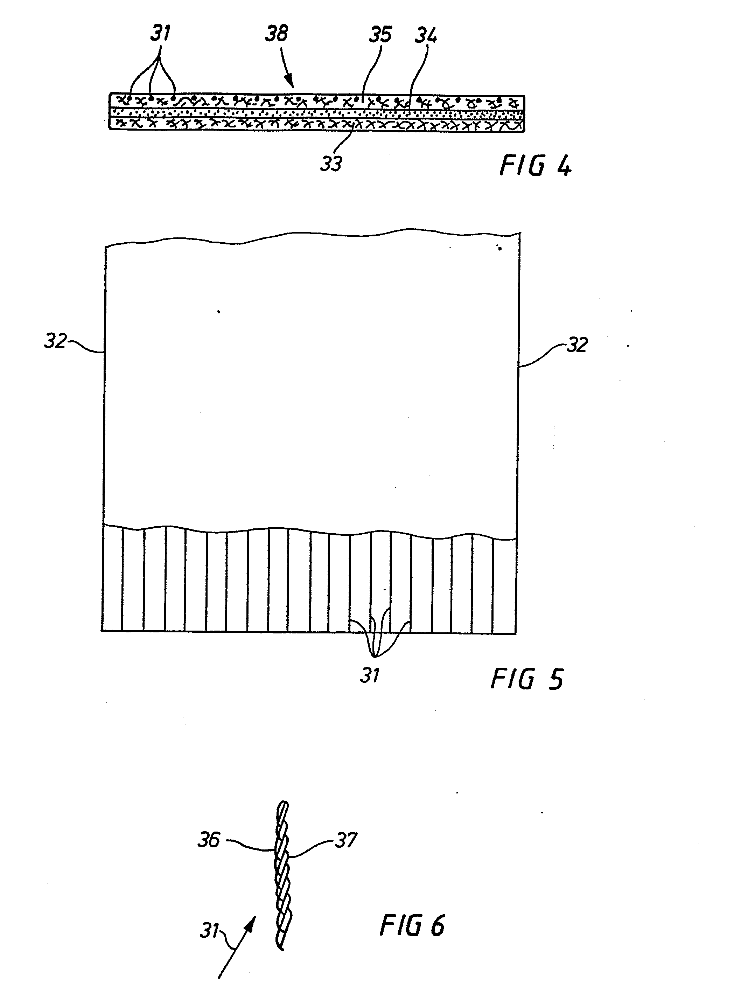

In der Figur 4 und 5 ist ein Unterbett 38 dargestellt; dabei wird die eine Außenschicht 33 von einem Schafwirkpelz gebildet, der aus 100%-iger Schafschurwolle gewirkt ist. Als nächstes ist dann ein Wollvlies 34 vorgesehen, welches ebenfalls aus 100%-iger Schafschurwolle besteht. Die andere Außenschicht wird von einem Trikotstoff 35 gebildet, bei dem im Ausführungsbeispiel Kupferfäden 31 eingewirkt bzw. eingewebt sind, die parallel zu den Längsseiten 32 des Unterbettes verlaufen.
In Figur 6 ist gezeigt, daß ein Kupferfaden 31 von mehreren, sehr dünnen, Kupferfäden 36,37, die miteinander verdrillt sind, gebildet wird.

Im Ausführungsbeispiel sind Kupferfäden mit 0,8 mm Durchmesser verwandt, die mit 10 mm Abstand voneinander angeordnet sind. Selbstverständlich können diese Abmaße in gewissen Grenzen variieren.

Der Textilstoff muß nicht nur als Anwendung bei einem Unterbett oder bei einem Kleidungsstück verwandt werden, sondern er kann überall dort verwandt werden, wo man Erdstrahlen abschirmen will, unabhängig davon, ob ein menschlicher Körper bzw. tierischer Körper abgeschirmt

werden soll.

P a t e n t a n s p r ü c h e

1. Betteinlage zur Abschirmung elektromagnetischer Felder im Schlafbereich, wobei die aus textilen sowie aus elektromagnetisch wirksame Materialien bestehende Betteinlage über oder unter der Matratze angeordnet oder als Zudecke ausgebildet ist, d a d u r c h g e k e n n z e i c h n e t , daß die Betteinlage eine von Schaumkunststoff umschlossene Metallfolie aufweist, welche sich über die gesamte Fläche der Betteinlage erstreckt, und daß die Metallfolie (5-8) eine hochpermeable Nickellegierungsfolie (Mu-Metall) ist.

2. Betteinlage nach Anspruch 1, d a d u r c h g e k e n n z e i ch n e t , daß die Metallfolie (5-8) eine Dicke von 0,05 bis 0,1 mm aufweist.

3. Betteinlage nach Anspruch 1, d a d u r c h g e k e n n z e i c h n et , daß die Metallfolie (5-8) in sich über die Breite der Betteinlage (1) erstreckende Streifen aufgeteilt ist, daß die Streifen in unterschiedlichen horizontalen Ebenen (19,20) in der Betteinlage angeordnet ist, und daß die Streifen der unterschiedlichen Ebenen (19,20) sich schuppenartig überlappen.

4. Betteinlage nach einem der Ansprüche 1 - 3, d a d u r c h g e k e n n z e i c h n e t , daß die Streifen der Metallfolie (5-8) jeder Ebene (19,20) in den Taschen (17,18) jeweils eines Tunnelgewebes (14,16) angeordnet sind.

5. Betteinlage nach Anspruch 4, d a d u r c h g e k e n n z e i c h n e t , daß die Metallfolien (5-8) zur Oberseite und Unterseite, sowie im Zwischenraum zwischen den horizontalen Ebenen (19,20) durch jeweils eine Schaumstoffschicht (2-4) abgedeckt ist.

6. Betteinlage zur Abschirmung elektromagnetischer Felder im Schlafbereich, wobei die aus textilen sowie aus elektromagnetisch wirksame Materialien bestehende Betteinlage über oder unter der Matratze angeordnet oder als Zudecke ausgebildet ist, d a d u r c h g e k e n n z e i c h n e t , daß eine Außenschicht (3) von einem Schafwirkpelz gewirkt aus 100%-iger Schafschurwolle besteht, darüber ein Wollvlies (4) aus 100%-iger Schafschurwolle folgt und als andere Außenschicht ein Trikotstoff (5) aus 100%-iger Baumwolle vorhanden ist, in den Kupferfäden (1) voneinander isolierend, z.B. durch Einwirken angeordnet sind.

7. Betteinlage nach Anspruch 6, d a d u r c h g e k e n n z e i c h n e t , daß die Kupferfäden (1) zueinander parallel verlaufend eingewebt und/oder eingewirkt sind.

8. Betteinlage nach Anspruch 7, d a d u r c h g e k e n n z e i c h n e t , daß die Kupferfäden (1) zu den Längsseiten (2) des Unterbettes parallel verlaufen.

9. Betteinlage nach Anspruch 6 - 8, d a d u r c h   g e k e n n z e i c h n e t , daß die Kupferfäden (1) einen Durchmesser von 0,6 bis 1,0 mm besitzen und im Abstand von 5 bis 15 mm voneinander angeordnet sind.

10. Betteinlage nach Anspruch 6,7 und 9 , d a d u r c h g e k e n n z e i c h n e t, daß der Kupferfaden (1) von mindestens zwei miteinander verdrillten Kupferfäden (6,7) gebildet ist.

11. Betteinlage nach einem der Ansprüche 6 - 10, d a d u r c h   g e k e n n z e i c h n e t , daß in der Verwendung für ein Kleidungsstück die Kupferfäden (1,6,7) zueinander parallel und voneinander isoliert verlaufend angeordnet sind.

FIG 1

0151414
1/4

0151414

FIG 2

SCH 603

0151414

FIG 3

SCH 603

FIG 4

FIG 5

FIG 6

SCH 603